# EUROPEAN PATENT APPLICATION

(11) **EP 1 589 119 A1**
(43) Date of publication of application: **26.10.2005**
(21) Application number: 04076193.4
(22) Date of filing: 19.04.2004
(51) Int. Cl.: C12Q 1/70

(54) **Detection of enterovirus RNA and method for reverse transcribing RNA**

(71) Applicant: Academisch Medisch Centrum, 1105 AZ Amsterdam (NL)
(72) Inventor: Beld, Marcellinus Gualbertus Hubertus Maria, 1187 JW Amstelveen (NL); Boom, Willem René, 1098 RL Amsterdam (NL); Sol, Cornelis Johannes Andreas, 1098 LR Amsterdam (NL)
(74) Representative: Winckels, Johannes Hubertus F.

(57) **Abstract**

Provided are means and methods for efficiently reverse transcribing RNA obtained from a variety of sources and especially from faeces. The invention further provides means and methods for efficiently and specifically detecting enteroviruses and particularly human enteroviruses in a sample.

## Description

The invention relates to the fields of diagnostics and the copying and/or amplification of nucleic acid. In particular, the invention relates to means and methods for detecting enterovirus using molecular biological tools.

The present invention is exemplified by means of human enteroviruses. However, the invention is by no means limited to enterovirus detection. Means and methods for isolating and reverse transcribing RNA are applicable using any type of RNA.

Human enteroviruses (EV) are members of the family Picornaviridae, are ubiquitous, and are mainly enterically transmitted. Enteroviruses have traditionally been identified by serotype-specific antisera in a virus-neutralizing test and 66 EV types are known to infect humans (20). Sixty-six EV serotypes were initially recognized and divided into 5 major groups: poliovirus (PV's; 1 to 3), Coxsackieviruses A (CAV's; 1 to 22 and 24), Coxsackieviruses B (CBV's; 1 to 6), echoviruses (1 to 7, 9, 11 to 27, and 29 to 33), and enteroviruses 68 to 71 (18). Recent molecular analyses have proven that echovirus 22 and 23 were genetically distinct from the members of the Enterovirus genus and were reclassified in a separate genus of Parechoviruses within the family of Picornaviridea (14, 21, 25).
Infections with EV cause a wide range of clinical outcomes like asymptomatic infections, aseptic meningitis (meningeal inflammation in the absence of a bacterial pathogen), encephalitis, paralytic poliomyelitis, and myocarditis. Although the majority of EV infections do not cause significant disease, infection can cause serious illness, especially in infants and immune-compromised patients. EV infections are the most common cause of aseptic meningitis and account for 80 - 90% of all cases of CNS infection for which a possible causative agent is identified (26). In the neonate, aseptic meningitis induced complications and poor outcome of EV infections generally occur within the first 2 days of life (1, 3). Aseptic meningitis in immune-competent adults is characterized by sudden onset of fever but neurological abnormalities are rare and both short term and long term outcome are generally good. Encephalitis caused by EV infections is a less common but a more severe disease than aseptic meningitis (19, 31, 32). Immune-compromised children and adults who are infected with EV may develop chronic meningitis and encephalitis, which may last years before becoming fatal (17).
Early clinical symptoms of meningitis caused by viruses, bacteria, and fungi are quite similar and difficult to distinguish but diagnosis, therapy, and outcome of disease caused by these pathogens vary considerably. Reliable laboratory diagnosis for EV is needed since specific and rapid diagnosis of EV meningitis has a significant impact on patient's management (11). Although, RT-PCR may become the diagnostic method of choice for EV infections of CNS, in many laboratories diagnosis of EV still relies on cell culture techniques. Stool specimens or rectal swabs, throat swabs, and CSF are used in virus culture. However, EV can be detected by RT-PCR in all kinds of clinical specimens like whole blood, plasma, serum, CSF, Stool specimens, throat swabs, vesicle fluids, pleuratic fluids, broncheo-alveolar-lavages, amniotic fluids, urine, and brain biopsies. Early in the acute phase of the illness, EV is frequently isolated from the throat whereas isolation of virus from CSF provides the most direct link to disease but is usually less successful. Cytopathic effect (CPE) is not recognizable within a few days and some enteroviruses do not grow in cell culture and therefore do not cause CPE at all (16, 27). In recent years, several EV RT-PCR assays have been developed which are sensitive and rapid (9, 10, 15, 27, 28). Although these methods improved the sensitivity and speed of the detection of enteroviruses, it was found that the sensitivity can be further improved by means and method of the present invention by 40%, range 25-55% (Table 5)

The invention therefore provides a method for reverse transcribing an RNA target molecule comprising incubating said RNA target molecule with a reverse transcriptase. Preferably, the reverse transcriptase is a Moloney Murine Leukemia Virus Reverse Transcriptase [M-MLV RT], more preferably a reverse transcriptase known as SuperScript II or III (RNase H- Reverse Transcriptase) described in Potter et al, Focus Vol 25.1; pp 19-24. SuperScript II contains a series of point mutations in the Rnase H domain of M-MLV, or a functional part, derivative and/or analogue of said SuperScript II or III, in a solution comprising between 2,5 and 7,5 mM of a suitable salt, preferably 5.0 mM, between 0.05 % and 0.2 % of a non-ionic detergent, preferably 0.1%, between 60 and 240 µM per nucleotide, preferably 120 µM and a suitable buffer that buffers said solution at a pH between about 8 or 10, said method further comprising incubating said solution at a temperature of between 25 and 50 °C.
A functional part, derivative and/or analogue of superscript II or III comprises the same activity in kind not necessarily in amount as superscript II or III themselves under the above mentioned conditions. Superscript and suitable parts, derivatives and analogues are described in Potter J. et al (2003): Focus, Vol 25.1, pp 19-24). Suitable salts are salts of group I of the periodic system, preferably a sodium or potassium salt. The counter-ion in the salt is preferably chloride. The non-ionic detergent is non-denaturing and significantly improves both the yield and quality of the reverse transcriptase (RT) product. The non-ionic detergent is preferably non-idet p40, or triton X100, or a combination or an equivalent thereof. Preferably the non-ionic detergent is Triton X100 or an equivalent thereof. The non-ionic detergent is preferably present in amount of between about 0.05 and 0.2 % (vol/vol). Preferably the non-ionic detergent is present in an amount of about 0.1% (vol/vol). Preferably, all four of different categories of nucleotides characterised by dATP, dCTP, dGTP and dTTP are added to the solution. One or more of the categories may be omitted, for instance when a specific typically short RT product is desired. However, at least one of the above mentioned categories is required in the solution. Currently, many different derivatives and analogues of the prototype nucleotides are available. Such derivatives and analogues, when they can be incorporated into the nascent strand, can also be used in the present invention. It is also possible to add one or more so-called stopper nucleotides in the solution for, for instance, sequencing purposes. In general any nucleotide or derivative and/or analogue thereof can be used in the present invention. A particular category of nucleotide is typically present in an amount of between about 60 and 240 µM per nucleotide. Preferably, a particular category of nucleotide is present in an amount of between about 100 and 140 µM, more preferably in an amount of about 120 µM. The temperature range is typically a range wherein superscript is active. Preferably, the temperature range is between about 25 and 50°C. Preferably, between about 40 and 50°C. More preferably, the incubation temperature is about 42 °C for Superscript II or a functional part, derivative and/or analogue thereof and 50 for Superscript III or a functional part, derivative and/or analogue thereof. The buffer can be any compound when it is suitable for use in enzymatic processes and capable of buffering a solution between about pH 8 and 10. Preferably, buffers common in enzymatic processes in molecular biology are used. Preferably the buffer comprises TRIS or HEPES or a combination or equivalent thereof. Preferably, the solution is buffered by said buffer at a pH of between about 8 and 10. Preferably between about pH 8 and 9. Preferably, at an pH between about 8.1 and 8.5. More preferably the solution is buffered by said buffer at an pH of about 8.3. Counter ions for adjusting the buffer to the desired pH are preferably sodium, potassium and chloride. For efficient reverse transcription to take place it is important that the reverse transcriptase primes efficiently. Efficient priming requires, as yet, the presence of a primer for the reverse transcriptase. The RNA itself can act as a primer as, for instance, is the case with genomic retroviral RNAs. However, typically at least one primer is added to the solution. The primer can be any oligonucleotide or equivalent thereof that is capable of hybridising to the RNA of interest and capable of being elongated by the reverse transcriptase. An equivalent of a primer oligonucleotide for RT priming comprises the same hybridisation and RT priming capability in kind not necessarily in amount. The primer oligonucleotide typically comprises between about 4 and 30 nucleotides or equivalent thereof. For random priming, the primer typically contains between about 4 to 10 nucleotides or equivalent thereof. For specific priming the primer typically contains between about 10 and 30 nucleotides or equivalents thereof, more preferably between about 15 and 25 nucleotides, preferably between about 18 and 22 nucleotides, more preferably about 20 nucleotides.
In a particularly preferred embodiment of random priming the primer is a hexamer.. For random priming it is preferred that the primer is a mixture of at least 5 and preferably at least 10 and more preferably at least 20 oligonucleotides or equivalent thereof, wherein said oligonucleotides or equivalents thereof differ from each other by at least one nucleotide. In a particularly preferred embodiment the primer is a hexamer mixture comprising at least 20 of the mentioned different oligonucleotides or equivalents thereof.
The RNA may be derived from any sample. However, it was found that the incubation conditions for reverse transcribing RNA are more robust than other conditions and allow for efficient reverse transcription of input RNA also when samples are obtained from notoriously difficult sources such as faeces. Thus in a preferred embodiment the invention provides a method of the invention further comprising preparing RNA target molecule from a sample comprising faeces, preferably human faeces.

In one aspect of the invention, the RNA target molecule comprises sequences derived from an enterovirus, preferably a human enterovirus (EV). Until recently the presence or absence of EV in faeces was analysed using cell lines to propagate any virus from the faeces. Of late, molecular diagnostics have been developed based on for instance amplification of enteroviral nucleic acid. Thus in one embodiment a method of the invention further comprises amplifying produced RT product using one or more primers. In previous studies, EV-RNA was purified without a control (IC-RNA) that monitored both RNA extraction efficiency and RT-PCR efficiency. In molecular diagnostics, the use of an IC-RNA is used for a reliable interpretation of results because it enables the verification of the sensitivity of the assay and avoids false negative results. Thus in one embodiment a method of the invention further comprises providing a sample containing the RNA target molecule with an internal control nucleic acid. Preferably this internal control nucleic acid comprises RNA. This may be done by adding the internal control to the sample or by adding the internal control already at an earlier stage, for example to the faeces or the isolated RNA there from. Thus in a preferred embodiment said solution further comprises an internal control RNA molecule. In vitro transcribed RNA can be used as an IC-RNA but is prone to degradation by RNAses. Packaging of IC-RNA into a phage protects the RNA from enzymatic degradation (24). These armoured RNA's can be spiked into clinical specimens without degradation, thus enabling simultaneous monitoring of the complete nucleic acid extraction and amplification process for each specimen. Thus in a preferred embodiment the internal control nucleic acid comprises armoured RNA. In a preferred embodiment the armoured RNA is prepared using the means and methods disclosed herein or disclosed in (24). Preferably the armoured RNA comprises a sequence 5'-CCC TGA ATG CGG CTA ATC CTA ACC ACG GAA CAG GCG GTC GCG AAC CAG TGA CTG GCT TGT CGT AAC GCG CAA GTC TGT GCT TGA GAC GTG CGT GGT AAC CGT CCG TGT TTC CTG TTA TTT TTA TCA TGG CTG CTT ATG GTG ACA AT-3'.

Like the wild-type EV target, IC-RNA's should have the same length, contain the same primer binding sites, and have the same GC content for identical extraction and amplification efficiency but should contain a different probe binding site for the differential detection of IC-RNA. Thus in a preferred embodiment the internal control nucleic acid comprises the same length, primer binding site(s) and GC content as the region in the EV RNA that is selected for reverse transcription and subsequent amplification. To discriminate between target and internal control said internal control comprises at least one location where the nucleic acid encodes a sequence that differs from at least all human enteroviruses. Detection of this region, for instance, by means of a probe thus can distinguish between internal control and EV-RNA. In a preferred embodiment the region that is selected for amplification comprises sequences that are conserved between human enteroviruses as this allows the use of one primer set for the detection of all (currently identified) enteroviruses. Preferably the selected region further comprises, a further location that is conserved between human enteroviruses, wherein the location is internal to the amplified region and does essentially not exhibit overlap with the location of the primer(s). Such a further conserved region allows the use of one detection means such as a probe for the detection of essentially all (currently known) human enteroviruses. Thus in one particularly preferred aspect the invention provides a method wherein said internal control RNA molecule comprises a sequence 5'-CCC TGA ATG CGG CTA ATC CTA ACC ACG GAA CAG GCG GTC GCG AAC CAG TGA CTG GCT TGT CGT AAC GCG CAA GTC TGT GCT TGA GAC GTG CGT GGT AAC CGT CCG TGT TTC CTG TTA TTT TTA TCA TGG CTG CTT ATG GTG ACA AT-3'. The primer(s) for amplification of this sequence allows amplification of essentially all human enteroviruses, whereas it further span a region that further comprises a further conserved region for the easy detection of amplified material from all, currently known, human enteroviruses. This sequence, and in particular the choice of the primer binding sites has the additional advantage that, whereas assay in the art detect also human viruses related to the human enteroviruses, the assay of the invention is more selective as at least the closely related human rhinoviruses are not detected using an assay of the invention. This feature of course greatly improves the reliability of the assay of the invention.
Thus in a particularly preferred embodiment the invention further provides a method for testing a sample for the presence therein of a nucleic acid derived from an enterovirus, comprising providing a nucleic acid amplification solution with said sample, with a first primer comprising a nucleotide sequence ID#1: 5'-CCC TGA ATG CGG CTA AT-3'; nucleotide positions [nt] 452-468) or a functional equivalent thereof and with a second primer comprising a nucleotide sequence (ID#2: 5'-ATT GTC ACC ATA AGC AGC C-3' nt 579-597 or a functional equivalent thereof, and incubating said amplification solution to allow for amplification of said nucleic acid when present. A functional equivalent of primer ID#1 or primer ID#2 comprises the same 10 bases when measured from the 3'-end but contains a mismatch of preferably 2, and more preferably 1 nucleotide in the 10 bases when measured from the 5'-end. It has been found that such functional equivalents comprise essentially the same specificity and amplification efficiency as the original they are derived from. Said sample preferably comprises product of a method for reverse transcribing an RNA target molecule according to the invention. In a preferred embodiment a method of the invention further comprises analysing the presence or absence of said nucleic acid by means of a probe. Preferably the presence of nucleic acid derived from a (human) enterovirus is analysed with a probe comprising a sequence ID#3: EV-specific probe; 5'-GCG GAA CCG ACT ACT TTG GGT-3' or a functional equivalent thereof. In a preferred embodiment a method for testing a sample of the invention further comprises analysing the presence or absence of an internal control nucleic acid by means of a probe comprising a sequence ID#4: IC-specific probe; 5'-CTT GAG ACG TGC GTG GTA ACC-3 or a functional equivalent thereof.

For detection probes may be labelled by any means suitable. Many different methods are known in the art. Amplified product can be measured directly or indirectly through measurement of label associated with the probe. Amplified product may be measured at the end of the amplification step or in real-time during amplification. Such methods are known in the art. When measured in real time, a probe preferably comprises a fluorophore. Other preferred labels include radio-active and/or digoxigenine labels. Label may be associated directly to the probe, or the probe may be detected indirectly, though a labelled binding member specific for said probe.

In another aspect the invention provides an aqueous solution for reverse transcribing an RNA target molecule comprising between 25 and 75 mM of a suitable salt, between 0.05 % and 0.2 % of a non-ionic detergent and a suitable buffer that buffers said solution at a pH between about 8 or 10. Preferably said solution further comprises between 60 and 240 µM nucleotides or equivalents thereof and a reverse transcriptase mentioned above or a functional part, derivative and/or analogue thereof. The invention also provides a concentrate of an aqueous solution of the invention. Preferably the concentrate comprises between 5 and 10 times the concentration of the chemicals mentioned for the aqueous solution of the invention.

In another aspect the invention provides an oligonucleotide comprising a sequence ID#1: 5'-CCC TGA ATG CGG CTA AT-3'; nucleotide positions [nt] 452-468; ID#2: 5'-ATT GTC ACC ATA AGC AGC C-3' nt 579-597; ID#3: EV-specific probe; 5'-GCG GAA CCG ACT ACT TTG GGT-3'; ID#4: IC-specific probe; 5'-CTT GAG ACG TGC GTG GTA ACC-3' or internal control 5'-CCC TGA ATG CGG CTA ATC CTA ACC ACG GAA CAG GCG GTC GCG AAC CAG TGA CTG GCT TGT CGT AAC GCG CAA GTC TGT GCT TGA GAC GTG CGT GGT AAC CGT CCG TGT TTC CTG TTA TTT TTA TCA TGG CTG CTT ATG GTG ACA AT-3' The invention further comprises a recombinant virus particle comprising a sequence (control probe or the complement thereof). Preferably, said recombinant virus particle is a recombinant phage particle. Preferably a phage having, expect for the nucleotide sequence of the region to be amplified using the oligonucleotide of the invention, the characteristics and elements of a phage as described in (24).

The invention further provides a kit for detecting enteroviral RNA comprising at least one and preferably at least two, more preferably at least three oligonucleotides of the invention. Preferably, the kit further comprises an internal control nucleic acid of the invention. Preferably, said kit comprises an armoured RNA according to (24) or the example herein, comprising an internal control sequence of the invention, preferably comprising a sequence 5'-CCC TGA ATG CGG CTA ATC CTA ACC ACG GAA CAG GCG GTC GCG AAC CAG TGA CTG GCT TGT CGT AAC GCG CAA GTC TGT GCT TGA GAC GTG CGT GGT AAC CGT CCG TGT TTC CTG TTA TTT TTA TCA TGG CTG CTT ATG GTG ACA AT-3' . Preferably, a kit of the invention comprises ID#4: IC-specific probe; 5'-CTT GAG ACG TGC GTG GTA ACC-3' and/or the complement thereof.. In a preferred embodiment the kit further comprises an aqueous solution of the invention and/or concentrate thereof. Preferably said kit further comprises superscript.

### Examples

### Materials and Methods

### Chemicals and enzymes.

Lysis buffer, wash buffers, and silica suspension were prepared as described previously (4). SuperScript II (SS II) was obtained from Life Technologies (Gaithersburg, MD). RNAse Inhibitor (RNAsin) was obtained from Promega, Madison, WI). Bovine serum albumin (BSA) was obtained from Roche Diagnostics (Almere, The Netherlands). Deoxynucleotides (dATP, dCTP, dGTP, dUTP), *Taq* DNA polymerase (Amplitaq Gold), uracil-N-glycosylase (Amperase) were from Applied Biosystems (Nieuwerkerk a/d IJssel, The Netherlands). Streptavidin-coated magnetic beads (Dynabeads M-280) were from Dynal (Hamburg, Germany). Tris, KCl, MgCl₂, Calf Thymus (CT)-DNA, were obtained from Sigma (Zwijndrecht, The Netherlands. Serum and plasma samples were obtained in Vacutainer tubes (Becton Dickinson Systems, Meylan, France).

### Clinical specimens.

In total 322 clinical specimens, including 281 CSF samples, 18 faeces samples, 10 throat swap samples, 3 vesicle fluids, 3 pleuratic fluids, 2 broncheo-alveolar-lavages, 2 amniotic fluids, 1 urine, and 2 brain biopsies, were obtained from patients suspected to be infected with EV and tested by the RT-PCR.

### Cytopathologic effect (CPE) for EV infection.

Viral culture was done by cocultivation of CSF with human diploid fibroblasts, tertiary monkey kidney cells, or Vero cells. These viral cultures were examined twice weekly for the appearance of EV-specific CPE. Preliminary identification of isolates was performed by either unstained CPE or by CPE incubated with specific Mab (DAKO-Enterovirus, 5-D8/1 [DAKO, Glostrup, Denmark]).

### Serotyping of isolates.

Typing of EV's was performed at the National Institute of Public Health and the Environment by neutralization tests with antiserum pools. These pools are prepared according to a combination so designed that an isolate can be screened for identity using 8 pools of 42 anti-sera in a single test.

### Viral strains.

The EV serotypes CVA [1-6, 18-22, 24], CVB [1-6], Echovirus [1-9, 11-22, 24-27, 29-33], Enterovirus [68-71], PV vaccines [1-3]), and Parechovirus 1 and 2 were kindly provided by the National Institute of Public Health and the Environment (Bilthoven, The Netherlands). The rhinovirus serotypes (1A, 1B, 3, 8, 11, 13, 14, 15, 16, and 88) were kindly provided by the Department of Virology from the Utrecht Medical Center (Utrecht, The Netherlands). The HAV serotype (HM175) was kindly provided by the Municipal Health Service Amsterdam.

### Primers.

Reverse transcription used random hexamers (Roche Diagnostics, Almere, The Netherlands), which were diluted in TE buffer (10 mM Tris-HCl, 1mM EDTA, pH 8.0) to 1.5 µg/µL. PCR primers were designed using computer-assisted analysis (OMIGA, Oxford Molecular, England) of all available 5' non-coding regions and full genomes of EV serotypes. HPLC-purified PCR primers were from Applied Biosystems (Nieuwerkerk a/d IJssel, The Netherlands) and were diluted in TE buffer to 100 ng/µL. Primers for amplification of both wild type EV RNA and IC RNA are located in the conserved 5' non-coding region of EV. The primer pair used for amplification consisted of entero-1 (ID#1: 5'-CCC TGA ATG CGG CTA AT-3'; nucleotide positions [nt] 452-468) and Bio-entero-2 (ID#2: 5'-ATT GTC ACC ATA AGC AGC C-3', 5' biotinylated; nt 579-597). Nucleotide numbering was according to the Sabin polio type 2 strain as described by Toyoda et al. (30).

### Armoured EV-RNA control.

The armoured EV-RNA control, containing part of the 5' non-coding region (nucleotide 428-691) was obtained from Ambion (Ambion, Inc., RNA diagnostics, Austin, Tx, USA) and was constructed according to the armoured RNA technique (24). The method is based on the packaging of recombinant RNA into MS2-like particles, which are produced in *Escherichia coli.* The particles are isolated through a series of conventional protein purification procedures. According to Ambion, the approximate conversion factor from 1 mg of armoured RNA to copies of RNA is about 2 x 10¹⁴. The armoured EV-RNA stock solution used in this paper with lot no. 040D49012A contained approximately 6.5 x 10¹⁴ EV-RNA c/mL. **Construction of armoured IC-RNA control.** For the construction of IC-RNA 2 oligonucleotides were designed by us and synthesized by Applied Biosystems (Ent-hyb-3; 5'- *CCC TGA ATG CGG CTA ATC* CTA ACC ACG GAA CAG GCG GTC GCG AAC CAG TGA CTG GTC TGT CGT AAC GCG CAA GTC TGT GCT TGA GAC GTG-3', and Ent-hyb-4; 5*'-ATT GTC ACC ATA AGC AGC* CAT GAT AAA AAT AAC AGG AAA CAC GGA CGG TTA CCA CGC ACG TCT CAA GCA CAG ACT T-3'). These 2 oligonucleotides, which together represent the same part of the 5' non-coding region (nucleotide 428-691) as present in the armoured EV-RNA control, were overlapping over a stretch of 20 nucleotides (underlined) and contained the same primer binding sites as the armoured EV-RNA control (in *Italics*) but with a different probe region (in bold). This IC-probe region allowed for discrimination between EV and IC RNA amplimers after hybridisation and detection. IC-RNA control was constructed by hybridisation and elongation of 1 ng Ent-hyb-3 and 1 ng Ent-hyb-4, 2.5 U of Amplitaq Gold, 5 µg of BSA, 1 x PCRII buffer (10 mM Tris-HCI; pH 8.3, 50 mM KCl), dATP, dCTP, dGTP, and dTTP at a concentration of 200 µM each, and 3 mM MgCl₂. The mixture was incubated for 10 min at 95°C, 5 min at 55°C, and 10 min at 72°C. The resulting hybrid was subsequently amplified with primer pair entero-1 and non-Bio-entero-2 in the same mixture as described above and was incubated 10 min at 95°C, followed by 35 cycles each consisting of 20 s at 95°C, 20 s at 55°C, and 1 min at 72°C, followed by 5 min at 72°C. The concentration of resulting amplimer was estimated by measuring the UV-absorption at 260 nm and subsequently 2 ng was cloned into a plasmid vector (PCRII, Promega, Ma) resulting in plasmid pEntIC 2. The IC-RNA sequence was confirmed by dideoxynucleotide sequencing (Visible Genetics Inc., Toronto, Canada). Packaging of IC-RNA control into MS2-like particles was performed with plasmid pEntIC 2 as the template and was custom made by Ambion. According to Ambion, the approximate conversion factor from 1 mg of armoured RNA to copies of RNA is about 2 x 10¹⁴. The armoured IC-RNA stock solution used in this paper with lot no. 021D49013A contained approximately 5.4 x 10¹⁴ IC-RNA c/mL.

### Dilution buffer for armoured RNA controls.

The armoured RNA controls were diluted in TSM dilution buffer containing 20 ng/µL CT-DNA, 10 mM Tris (pH 7.5), 100 mM NaCl, 1 mM MgCl₂, 0.1% gelatin (Sigma, catalogue no. G-9382) and stored at -20°C. Final solution of armoured EV-RNA contained 500 copies/µL and the final solution of armoured IC-RNA contained 100 copies/µL.

### RNA purification.

EV-RNA was purified from clinical specimens as described earlier (5), with the following modifications; 20 µL of size-fractionated silica particles was used in combination with 900 µL of lysis buffer L6. To this silicalysis buffer mixture, the clinical specimen and 500 armoured IC-RNA copies were added. RNA was eluted in 100 µL TE buffer.

### RT-PCR.

Forty µL of the 100 µL RNA eluate (2/5) was used for reverse transcription. The final reverse transcription mixture (50 µL) contained 1500 ng hexamers, 1 x CMB1 buffer (10 mM Tris-HCl; pH 8.3, 50 mM KCl, 0.1% Triton), 0.4 U/µL of SSII, 120 µM of each dNTP, 0.08 U/µL RNAsin, and 5 mM MgCl₂. The mixture was incubated for 30 minutes at 42°C and 25 µL (corresponding to 40 µL [1/5] of CSF) was subsequently used as input in the PCR. The PCR was performed in a 50 µL volume containing 200 ng of entero-1 (5'-CCC TGA ATG CGG CTA AT-3'; nucleotide positions [nt] 452-468) and 200 ng of Bio-entero-2 (5'-ATT GTC ACC ATA AGC AGC C-3', 5' biotinylated; nt 579-597), 0.05 U/µL of Amplitaq Gold DNA polymerase, 0.01 U/µL Amperase, 0.1 µg/µL of BSA, 1 x PCRII buffer (10 mM Tris-HCI; pH 8.3, 50 mM KCl), dATP, dCTP, and dGTP at a concentration of 200 µM each, and 400 µM dUTP. The final MgCl₂ concentration in PCR reaction was 2.5 mM. PCR's were performed in an Applied Biosystems 9600 thermocycler: 2 min at 50°C, 10 min at 95°C, followed by 45 cycles each consisting of 20 s at 95°C, 20 s at 55°C, and 1 min at 72°C, followed by 5 min at 72°C.

### Hybridisation and measurement by ECL.

After RT-PCR, excess primers were removed as earlier described (6), and amplicons were hybridised with TBR (Tris [2,2'-bipyridine] ruthenium [II] chelate)-labelled probes specific for EV-RNA and IC-RNA as recently described (6). Probes (nt 531-551) were 5' TBR-labelled and were as follows, TBR-entero-1 (ID#3: EV-specific probe; 5'-GCG GAA CCG ACT ACT TTG GGT-3' and TBR-entero-2 (ID#4: IC-specific probe; 5'-CTT GAG ACG TGC GTG GTA ACC-3'). Hybrids were captured with streptavidin-coated magnetic (beads and the electrochemiluminescence (ECL) signal, expressed in luminosity units (LU), was measured by the M8 system (IGEN, Oxford, England). In real time PCR this step can be omitted. With this device, a 96-well plate is used and unhybridised TBR-labelled probes are automatically removed by washing. The amount of labelled hybrids is determined after excitation by applying an electric field.

### Other labels can also be used, such as fluorophores in real-time PCT, radioactive labels, digoxigenine etc.

A signal of more than 500 LU (= 2.5-times mean background signal for either probe) was considered to be positive. A clinical specimen was considered positive for EV-RNA if more than 500 LU were measured with the EV probe, regardless of the result obtained for the IC probe. A clinical specimen was considered to be negative for EV-RNA if less than 500 LU were measured with the EV probe and IC- RNA was detected at more than 500 LU. In the diagnostic RT-PCR, 4 controls were included in RNA extraction; 2 positive controls and 2 negative controls. The high positive control (HPC) contained 12,500 armoured EV-RNA copies/extraction and the low positive control (LPC) contained 2500 armoured EV-RNA copies/extraction, both together with 500 armoured IC-RNA copies. The first negative control contained 500 armoured IC-RNA copies and served as a control for the entire procedure and should be negative for EV probe but positive for the IC probe. The second negative control contained no armoured EV-RNA copies or armoured IC-RNA copies and should be negative for both probes with a mean of 200 LU.

### Parechovirus specific RT-PCR.

The conditions of the RT-PCR reaction to detect Parechoviruses were similar as the above described protocol except for the primers used in PCR. For the specific detection of Parechoviruses we used the primer pair as described by Oberste et al. (22).

### Results

### Determination of the lower limit of detection of the EV RT-PCR assay.

To determine the lower limit of detection of the EV RT-PCR assay, we spiked decreasing amounts of both armoured EV-RNA and armoured IC-RNA diluted in TSM buffer into 200 µL of EV-negative CSF before RNA extraction with the Boom method (5). RNA was diluted in 100 µL and 40 µL of extracted RNA (2/5) was used in RT and subsequently 50% of the cDNA was used in PCR (1/5 of the extracted RNA). Limiting dilutions of the armoured EV-RNA revealed a detection limit of 84 EV-RNA copies in extraction with a 50% hit rate (5/10 runs) resulting in a detection limit of 17 copies of EV-DNA in PCR (1/5). Limiting dilutions of the armoured IC-RNA revealed a detection limit of 28 IC-RNA copies in extraction with a 60% hit rate (6/10 runs) resulting in a detection limit of 5 copies of IC-DNA in PCR (1/5) (Table 1). Identical results were found after the direct release of EV-RNA and IC-RNA from its phage (without CSF as background) after incubation at 70°C for 5 minutes (results not shown).

### Testing of EV serotypes.

All known EV serotypes (n = 64) were tested with the described EV RT-PCR. The extraction of every single EV serotype and non-enterovirus serotype was done alternated with negative extraction controls that contained IC-RNA. All EV serotypes were positive whereas the non-enteroviruses, including echovirus 22 and 23, rhinoviruses, HAV, and all negative controls were negative. The negative results were truly negative since the ECL signals for co-extracted armoured IC-RNA were all positive (Table 2).

### Testing of the 2001 and 2002 QCMD EV proficiency panels.

These proficiency panels from 2001 and 2002 consisted of coded freeze-dried samples, including an Echo 11 and CVA 9 dilution series, other EV serotypes representing different genetic clusters of human EV's, and negative controls. The freeze-dried samples were reconstituted with 1 mL of sterile water according to the QCMD protocol and 200 µL thereof was used in extraction and amplified as described in the materials and methods section. All samples of the 2001 QCMD proficiency panel were positive regardless of the serotypes and all included negative controls were truly negative, since the ECL signals for co-extracted armoured IC-RNA were all positive (Table 3).

### Testing of clinical specimens.

A total of 322 clinical specimens were obtained from patients suspected to be infected with EV and were tested by the RT-PCR. Forty-five positive results (14%), 267 negative results (82.9%), and 10 invalid results (3.1%) were found. These invalid results were found in 7/281 CSF samples (2.5%), in 2/18 faeces samples (11.1%), and in 1/10 throat swab samples (10%). Repeating these 10 invalid samples with the optimised RT-PCR revealed that all invalid results apart from 1 faeces sample were valid, negative for EV, and could be reported to the clinician. (Table 4).

### Comparison of virus culture with RT-PCR.

Eighty-seven clinical specimens suspected to be infected with EV were available to be tested in virus culture and in RT-PCR. Agreement between virus culture and RT-PCR was found in 72/87 clinical specimens (82.8%). In 54 clinical specimens a negative result was found for both virus culture and RT-PCR, whereas 18 clinical specimens were positive in virus culture and in RT-PCR. Discordant results were found in 15 clinical specimens. In 3 of them virus culture was positive whereas RT-PCR was truly negative for EV-RNA in RT-PCR since the results for IC-RNA were positive. Two of these RT-PCR negative samples were subjected to a specific Parechovirus RT-PCR and were found to be positive (results not shown). In the other RT-PCR negative sample CPE was only found after 4 weeks. The remaining discordant results consisted of 12 clinical specimens that were RT-PCR positive and negative in virus culture (Table 5).

### Discussion.

Isolation of EV in cell culture is still regarded as the diagnostic gold standard. There are however many disadvantages to culture such as its labour-intensity, the delay of days to weeks to obtain a positive result, and its false negativity rate of approximately 25-35% because of failures in antibody neutralization and the inability of certain CVA serotypes to grow in cell culture (16, 27). Many of the disadvantages of cell culture can be overcome by RT-PCR. Most frequently used primer and probe sets for the detection of EV are those described by Chapman et al. (10) and Rotbart et al. (27). These primers and probes have been proven to be reactive with all known EV and fail to amplify echovirus 22 and 23, which have been reported to be a genetic distinct genus in the family of Picornaviridae (14, 21, 25). We designed a primer and probe set with minor modifications in comparison to those first described by Chapman et al. and Rotbart et al. and found that not only all known EV serotypes would be detected but also that cross-reactivity between EV serotypes and rhinoviruses was avoided. This cross-reactivity with rhinoviruses has an impact if nasal or pharyngeal swab specimens are used for testing.

We have described a RT-PCR assay in a non-nested format for the detection of EV-RNA in clinical specimens in which 2500 c/mL of armoured IC-RNA mimicking the EV target were included in the RNA extraction and all subsequent steps of the procedure. This armoured IC-RNA can be spiked directly into clinical specimens without degradation of the RNA and enables monitoring of the complete nucleic acid extraction and amplification process for each specimen.

The sensitivity of the RT-PCR was evaluated with limiting dilutions of both armoured EV-RNA and armoured IC-RNA. We found a detection limit of 5 copies of IC-DNA in 60% of cases in PCR. Poisson statistics predicts that 63% of the reactions will be positive with a single copy of DNA in PCR (13). Thus, if RNA was extracted and reversed transcribed with 100% efficiency, the 60% detection rate should represent 1 copy of IC-DNA in PCR. Since we found comparable results after the direct release of RNA from the armoured RNA through incubation at 70°C for 5 minutes and after extraction of the RNA from the armoured RNA using the Boom method, our results might suggest a less efficient reverse transcription step or the presence of a certain percentage of empty armoured RNA phages. In addition, our observed approximately 3-fold difference in detection between EV-RNA and IC-RNA might be explained by inaccuracies in the determination of the concentration and dilution steps of the stock solutions. The lower limit of sensitivity of the RT-PCR was based on the lower detection rate of IC-RNA and was about 150 IC-RNA c/mL. The presence of 500 copies of IC-RNA during extraction from 200 µL of clinical specimens allowed us to draw the conclusion that a specimen found to be negative for EV-RNA but positive for IC-RNA would contain less than 2500 copies of EV-RNA/mL.

The use of the armoured IC-RNA was critical in the detection of false-negative reactions or invalid results. Currently, viral culture is still the gold standard to determine an EV infection. Validation of a RT-PCR assay for the diagnosis of EV infections is difficult because cases are defined clinically and viral culture has a poor sensitivity of approximately 70%, partly due to the inability of certain CVA serotypes to grow in cell culture (16, 27). We found an overall good agreement of 82.8% between virus culture and RT-PCR. CSF provides the most direct link to disease but is usually less successful in virus culture. We found high discordant results (17.2%) between virus culture and RT-PCR and mainly in CSF samples. Delayed processing and improper handling of the clinical specimens may have contributed to false negative results in virus culture and our results showed the added value of the described RT-PCR assay for the diagnosis of an EV-infection.

In conclusion, viral EV culture with a good clinical diagnosis will still be of use for EV diagnosis. However, the described EV RT-PCR assay will be of benefit as a sensitive, highly specific, faster assay for the detection of EV in clinical specimens, and the use of an armoured IC RNA strongly reduces false negative results.

### References.

1. **Abzug, M. J., M. J. Levin, and H. A. Rotbart**. 1993. Profile of enterovirus disease in the first two weeks of life. Pediatr.Infect.Dis.J. 12:820-824.
2. **Altman, D. G**. 1999. Inter-rater agreement, p. 403-405. In Chapman & Hall (ed.), Practical statistics for medical research. CRC, London.
3. **Berlin, L. E., M. L. Rorabaugh, F. Heldrich, K. Roberts, T. Doran, and J. F. Modlin. 1993**. Aseptic meningitis in infants < 2 years of age: diagnosis and etiology. J.Infect. Dis. 168:888-892.
4. **Boom, R., C. J. Sol, M. M. Salimans, C. L. Jansen, P. M. Wertheim-van Dillen, and J. van der Noorda. 1990.** Rapid and simple method for purification of nucleic acids. J.Clin.Microbiol. 28:495-503.
5. **Boom, R., C. Sol, J. Weel, K. Lettinga, Y. Gerrits, A. van Breda, and P. Wertheim-van Dillen. 2000.** Detection and quantitation of human cytomegalovirus DNA in faeces. J.Virol.Methods 84:1-14.
6. **Casas, I., P. E. Klapper, G. M. Cleator, J. E. Echevarria, A. Tenorio, and J. M. Echevarria. 1995.** Two different PCR assays to detect enteroviral RNA in CSF samples from patients with acute aseptic meningitis. J.Med.Virol. 47:378-385.
7. **Chapman, N. M., S. Tracy, C. J. Gauntt, and U. Fortmueller. 1990.** Molecular detection and identification of enteroviruses using enzymatic amplification and nucleic acid hybridization. J.Clin.Microbiol. 28:843-850.
8. **Chonmaitree, T., C. D. Baldwin, and H. L. Lucia. 1989.** Role of the virology laboratory in diagnosis and management of patients with central nervous system disease. Clin.Microbiol.R.ev. **2:1-14.**
9. **Chruscinska, E., M. Dyba, G. Micera, W. Ambroziak, J. Olczak, J. Zabrocki, and H. Kozlowski. 1997**. Inorg. Biochem. 66:19-22.
10. **Diaco, R. 1995**. Practical considerations for the design of quantitative PCR assays. p. 84-108. *In* M. A. Innis, D. H. Gelfland, and J. J. Sninsky (eds.), PCR strategies. Academic Press, Inc., New York.
11. **Hyypia, T., T. Hovi, N. J. Knowles, and G. Stanway. 1997.** Classification of enteroviruses based on molecular and biological properties. J.Gen.Virol. 78 (Pt 1):1-11.
12. **Kessler, H. H., B. Santner, H. Rabenau, A. Berger, A. Vince, C**. **Lewinski, B. Weber, K. Pierer, D. Stuenzner, E. Marth, and H. W. Doerr. 1997**. Rapid diagnosis of enterovirus infection by a new one-step reverse transcription-PCR assay. J.Clin.Microbiol. 35:976-977.
13. **Lipson, S. M., R. Walderman, P. Costello, and K. Szabo. 1988.** Sensitivity of rhabdomyosarcoma and guinea pig embryo cell cultures to field isolates of difficult-to-cultivate group A Coxsackieviruses. J.Clin.Microbiol. 26:1298-1303.
14. **McKinney, R. E., Jr., S. L. Katz, and C. M. Wilfert. 1987**. Chronic enteroviral meningoencephalitis in agammaglobulinemic patients. Rev.Infect.Dis. 9:334-356.
15. **Melnick, J. L. 1996**. Enteroviruses: Polioviruses, Coxsackieviruses, echoviruses, and newer enteroviruses., p. 655-712. *In* D. M. Knipe and P. M. Howley (eds.), Fields. Lippincott-Raven, Philadelphia.
16. **Modlin, J. F., R. Dagan, L. E. Berlin, D. M. Virshup, R. H**. **Yolken, and M**. **Menegus. 1991**. Focal encephalitis with enterovirus infections. Pediatrics 88:841-845.
17. **Muir, P., U. Kammerer, K. Korn, M. N. Mulders, T. Poyry, B. Weissbrich, R. Kandolf, G. M. Cleator, and A. M. van Loon. 1998.** Molecular typing of enteroviruses: current status and future requirements. The European Union Concerted Action on Virus Meningitis and Encephalitis. Clin.Microbiol.Rev. 11:202-227.
18. **Oberste, M. S., K. Maher, and M. A. Pallansch. 1998.** Complete sequence of echovirus 23 and its relationship to echovirus 22 and other human enteroviruses. Virus Res. 56:217-223.
19. **Oberste**, **M**. **S**., **K**. **Maher, and M**. **A**. **Pallansch. 1999**. Specific detection of echoviruses 22 and 23 in cell culture supernatants by RT-PCR. J.Med.Virol. 58:178-181.
20. **Pallansch, M. A. and R. P. Roos. 2001.** Enteroviruses: polioviruses, Coxsackieviruses, echoviruses and newer enteroviruses, p. 723-776. In D. M. Knipe and P. M. Howley (eds.), Fields. Lippicott Williams & Wilkins, Philadelphia.
21. **Pasloske, B. L., C. R. Walkerpeach, R. D. Obermoeller, M. Winkler, and D. B. DuBois. 1998.** Armored RNA technology for production of ribonuclease-resistant viral RNA controls and standards. J.Clin.Microbiol. 36:3590-3594.
22. **Poyry, T., L. Kinnunen, T. Hyypia, B. Brown, C. Horsnell, T. Hovi, and G. Stanway. 1996.** Genetic and phylogenetic clustering of enteroviruses. J.Gen.Virol. 77 (Pt 8):1699-1717.
23. **Rotbart, H. A., M. H. Sawyer, S. Fast, C. Lewinski, N. Murphy, E. F. Keyser, J. Spadoro, S. Y. Kao, and M. Loeffelholz. 1994.** Diagnosis of enteroviral meningitis by using PCR with a colorimetric microwell detection assay. J.Clin.Microbiol. 32:2590-2592.
24. **Rotbart, H. A. 1995.** Enteroviral infections of the central nervous system. Clin.Infect.Dis. 20:971-981.
25. **Stellrecht, K. A., I. Harding, F. M. Hussain, N. G. Mishrik, R. T. Czap, M. L. Lepow, and R. A. Venezia. 2000.** A one-step RT-PCR assay using an enzyme-linked detection system for the diagnosis of enterovirus meningitis. J.Clin.Virol. 17:143-149.
26. **Toyoda, H**., **M**. **Kohara**, **Y**. **Kataoka**, **T**. **Suganuma**, **T**. **Omata**, **N**. **Imura, and A. Nomoto. 1984**. Complete nucleotide sequences of all three poliovirus serotype genomes. Implication for genetic relationship, gene function and antigenic determinants. J.Mol.Biol. 174:561-585.
27. **Whitley, R. J. 1990.** Viral encephalitis. N.Engl.J.Med. 323:242-250.
28. **Whitley, R. J. and J. W. Gnann. 2002.** Viral encephalitis: familiar infections and emerging pathogens. Lancet 359:507-513.

**Table 1a.**

| Sensitivity for armored EV-RNA control phages. | | | |
|---|---|---|---|
| EV-RNA copies/200 µL CSF in extraction | in RT (2/5) | in PCR (1/5)* | proportion positive |
| 900 | 360 | 180 | 10/10(100%) |
| 450 | 180 | 90 | 9/10 (90%) |
| 225 | 90 | 45 | 8/10 (80%) |
| 112 | 45 | 23 | 8/10 (80%) |
| 84 | 34 | 17 | 5/10 (50%) |
| 56 | 23 | 11 | 6/20 (30%) |
| 28 | 11 | 6 | 5/16 (3%) |
| 14 | 6 | 3 | 1/10(10%) |
| 7 | 3 | 1 | 1/10 (10%) |
| 0 | 0 | 0 | 0/10 (0%) |

| | | | |
|---|---|---|---|
| *For the copy numbers presented in Table 1 the following assumptions were made; (i) The armoured RNA stocks contain the specified number of phage particles and all contain EV-RNA or IC-RNA, (ii) a 100% efficiency in extraction, in reverse transcription, and in PCR. | | | |

**Table 1b.**

| Sensitivity for armoured IC-RNA control phages. | | | |
|---|---|---|---|
| IC-RNA copies/200 µL CSF in extraction | in RT (2/5) | in PCR (1/5)* | proportion positive |
| 225 | 90 | 45 | 10/10 (100%) |
| 112 | 45 | 23 | 9/10 (90%) |
| 56 | 23 | 11 | 8/10(80%) |
| 28 | 11 | 6 | 6/10 (60%) |
| 0 | 0 | 0 | 0/10(0%) |

| | | | |
|---|---|---|---|
| *For the copy numbers presented in Table 1 the following assumptions were made; (i) The armoured RNA stocks contain the specified number of phage particles and all contain EV-RNA or IC-RNA, (ii) a 100% efficiency in extraction, in reverse transcription, and in PCR. | | | |

**Table 2.**

| Specificity of EV RT-PCR for prototype strains of enteroviruses and controls. | | | |
|---|---|---|---|
| Virus serotype | Source | Result | Mean LU EV-RNA (range) |
| CVA (2-4, 6-18, 20, 21, 24^{a}) | Cell culture | Pos | 87,000 (57,000-117,000) |
| CVA (1, 5, 19, 22) | Suckling mice | Pos | 87,000 (73,000-100,000) |
| CVB (1- 6) | Cell culture | Pos | 91,000 (75,000-107,000) |
| Echovirus (1-9, 11-16, 17-21,24-27,29-33^{b,c,d,e}) | Cell culture | Pos | 98,000 (86,000-111,000) |
| Echovirus (22, 23^{f}) | Cell culture | Neg | 218 (209-227) |
| Enterovirus (68-71) | Cell culture | Pos | 77,000 (68,000-86,000) |
| Poliovirus (1, 2, 3) | Cell culture | Pos | 104,000 (79,000-129,000) |
| Rhinovirus (1A, 1B, 3, 8, 11, 13, 14, 15, 16, 88) | Cell culture | Neg | 228 (205-277) |
| HAV | Cell culture | Neg | 237 |
| Negative control | | Neg | 226 (217-235) |
| HPC (25,000 EV-RNA c/mL) | | Pos | 85,000 (62,000-106,000) |
| LPC (5,000 EV-RNA c/mL) | | Pos | 58,000 (46,000-70,000) |
| IC (2,500 IC RNA c/mL) | | Pos | 51,000 (40,000-63,000) |

| | | | |
|---|---|---|---|
| ^{a} Echovirus 34 is a variant of CVA24. | | | |
| ^{b} Echovirus 8 is a variant of echovirus 1. | | | |
| ^{c} CVA23 is a variant of echovirus 9. | | | |
| ^{d} Echovirus 10 was reclassified as reovirus 1. | | | |
| ^{e} Echovirus 28 was reclassified as human rhinovirus 1A. | | | |
| ^{f} Echovirus 22 and 23 were reclassified as parechovirus types 1 and 2. Source: adapted from Melnick, Fields Virology, 3^{rd} edition, 1996 (18). | | | |

**Table 3.**

| Composition and results of the QCMD proficiency panel 2001. | | | | | |
|---|---|---|---|---|---|
| Code | Serotype | Virus titer* | LU EV-RNA | LU IC-RNA | Result |
| EV-C01 | CXVA 9 | 0.036 | 31,104 | 65,482 | Positive |
| EV-C02 | CXVA 9 | 0.36 | 87,325 | 81,793 | Positive |
| EV-C03 | CXVA 9 | 3.6 | 90,570 | 71,435 | Positive |
| EV-C04 | No virus | | 214 | 89,860 | Negative |
| EV-C06 | Echo 11 | 25.2 | 96,460 | 85,396 | Positive |
| EV-C11 | Echo 11 | 252 | 99,930 | 43,751 | Positive |
| EV-C09 | Echo 11 | 25,200 | 87,380 | 860 | Positive |
| EV-C07 | CXVB 5 | 317 | 83,867 | 32,092 | Positive |
| EV-C08 | EV 71 | 56.4 | 98,508 | 31,411 | Positive |
| EV-C10 | No virus | | 226 | 74,234 | Negative |
| EV-C05 | Echo 6 | 20,000 | 82,529 | 27,549 | Positive |

| | | | | | |
|---|---|---|---|---|---|
| *Titer of original virus stocks before inactivation and freeze-drying (TCID₅₀/mL): CVA 9; 3.6 x 10⁶, Echo 6; 2.0 x 10⁸, Echo 11; 2.5 x 10⁷, CVB 5; 3.2 x 10⁷, EV 71; 5.6 x 10⁶. | | | | | |

**Table 4.**

| Summary of results after testing clinical specimens. | | | |
|---|---|---|---|
| Specimen | N | Positive | Negative |
| CSF | 281 | 36 | 238 |
| Faeces | 18 | 6 | 10 |
| Throat swabs | 10 | 0 | 9 |
| Other | 13 | 3 | 10 |
| Total no. | 322 | 45 (14%) | 267 (82.9%) |

**Table 5.**

| Comparison of RT-PCR and virus culture on different clinical specimens obtained from patients with a clinically suspected EV infection. | | | | | |
|---|---|---|---|---|---|
| Clinical specimen | No tested | RT-PCR + Culture + | RT-PCR + Culture - | RT-PCR -Culture - | RT-PCR -Culture + |
| CSF | 67 | 15 | 10 | 40 | 2 |
| Faeces | 13 | 3 | 1 | 9 | 0 |
| Throat swap | 4 | 0 | 0 | 4 | 0 |
| Urine | 1 | 0 | 0 | 1 | 0 |
| Biopsy | 1 | 0 | 1 | 0 | 0 |
| Total no. | 86 | 18 (20.9%) | 12 (14.0%) | 54(62.8%) | 2* (2.3%) |

| | | | | | |
|---|---|---|---|---|---|
| * These culture isolates were identified as Parechovirus and not as Enterovirus. | | | | | |

## Claims

1. A method for reverse transcribing an RNA target molecule comprising incubating said RNA target molecule with a reverse transcriptase, preferably a moloney Murine Leukemia Virus reverse transcriptase or SuperScript II or SuperScript II or a functional part, derivative and/or analogue thereof in a solution comprising between 25 and 75 mM of a suitable salt, between 0.05 % and 0.2 % of a non-ionic detergent, between 60 and 240 µM nucleotides or equivalents thereof and a suitable buffer that buffers said solution at a pH between about 8 or 10, said method further comprising incubating said solution at a temperature of between 35 and 50°C.

2. A method according to claim 1, wherein the solution is buffered at a pH between about 8 and 9.

3. A method according to claim 1 or claim 2, wherein the solution is buffered at a pH between about 8.1 and 8.5.

4. A method according to claim 3, wherein the solution is buffered at a pH of about 8.3.

5. A method according to any one of claims 1-4, wherein said solution further comprises random hexamer primers.

6. A method according to any one of claims 1-5, wherein said RNA target molecule is derived from a faecal sample.

7. A method according to claim 6, wherein said RNA target molecule comprises an enterovirus.

8. A method according to any one of claims 1-7, wherein said solution further comprises an internal control RNA molecule.

9. A method according to claim 8, wherein said internal control RNA molecule comprises a sequence 5'-CCC TGA ATG CGG CTA ATC CTA ACC ACG GAA CAG GCG GTC GCG AAC CAG TGA CTG GCT TGT CGT AAC GCG CAA GTC TGT GCT TGA GAC GTG CGT GGT AAC CGT CCG TGT TTC CTG TTA TTT TTA TCA TGG CTG CTT ATG GTG ACA AT-3'

10. A method for testing a sample for the presence therein of a nucleic acid derived from an enterovirus, comprising providing a nucleic acid amplification solution with said sample, with a first primer comprising a nucleotide sequence ID#1: 5'-CCC TGA ATG CGG CTA AT-3'; nucleotide positions [nt] 452-468 or a functional equivalent thereof and with a second primer comprising a nucleotide sequence ID#2: 5'-ATT GTC ACC ATA AGC AGC C-3' nt 579-597 or a functional equivalent thereof, and incubating said amplification solution to allow for amplification of said nucleic acid when present.

11. A method according to claim 10, wherein said sample comprises product of a method for reverse transcribing an RNA target molecule according to any one of claims 1-9.

12. A method according to claim 10 or 11, further comprising analysing the presence or absence of said nucleic acid by means of a probe.

13. A method according to claim 12, wherein the presence of nucleic acid derived from an enterovirus is analysed with a probe comprising a sequence ID#3: EV-specific probe; 5'-GCG GAA CCG ACT ACT TTG GGT-3'or a functional equivalent thereof.

14. A method according to claim 12 or claim 13, further comprising analysing the presence or absence of an internal control nucleic acid by means of a probe comprising a sequence ID#4: IC-specific probe; 5'-CTT GAG ACG TGC GTG GTA ACC-3' or a functional equivalent thereof.

15. An aqueous solution for reverse transcribing an RNA target molecule comprising between 25 and 75 mM of a suitable salt, between 0.05 % and 0.2 % of a non-ionic detergent and a suitable buffer that buffers said solution at a pH between about 8 or 10.

16. A method according to any one of claim 12-14 wherein said product of said amplification is measured in real-time.

17. An aqueous solution according to claim 16, further comprising between 60 and 240 µM of at least one nucleotide or equivalents thereof and a reverse transcriptase, preferably a Moloney Murine Leukemia Virus reverse transcriptase or SuperScript II or SuperScript II or a functional part, derivative and/or analogue thereof.

18. A concentrate of an aqueous solution according to claim 16.

19. An oligonucleotide comprising a sequence
ID#1: 5'-CCC TGA ATG CGG CTA AT-3'; nucleotide positions [nt] 452-468;
ID#2: 5'-ATT GTC ACC ATA AGC AGC C-3' nt 579-597; ID#3: EV-specific probe; 5'-GCG GAA CCG ACT ACT TTG GGT-3'; ID#4: IC-specific probe; 5'-CTT GAG ACG TGC GTG GTA ACC-3' or internal control 5'-CCC TGA ATG CGG CTA ATC CTA ACC ACG GAA CAG GCG GTC GCG AAC CAG TGA CTG GCT TGT CGT AAC GCG CAA GTC TGT GCT TGA GAC GTG CGT GGT AAC CGT CCG TGT TTC CTG TTA TTT TTA TCA TGG CTG CTT ATG GTG ACA AT-3.

20. A recombinant virus particle comprising a sequence the sequence 5'-CCC TGA ATG CGG CTA ATC CTA ACC ACG GAA CAG GCG GTC GCG AAC CAG TGA CTG GCT TGT CGT AAC GCG CAA GTC TGT GCT TGA GAC GTG CGT GGT AAC CGT CCG TGT TTC CTG TTA TTT TTA TCA TGG CTG CTT ATG GTG ACA AT-3' and/or ID#4 5'-CTT GAG ACG TGC GTG GTA ACC-3' or the complement thereof.

21. A kit for detecting enteroviral RNA comprising at least one and preferably at least two, more preferably at least three oligonucleotides according to claim 19.

22. A kit according to claim 21, further comprising an internal control nucleic acid, comprising a sequence according to claim 18, or a functional equivalent thereof.

23. A kit according to claim 22, comprising an armoured internal control RNA.

24. A kit according to any one of claims 21-23, further comprising an aqueous solution according to claim 16 or claim 17 and/or a concentrate according to claim 18.

25. A kit according to any one of claims 21-24, further comprising SuperScript II or SuperScript III.


SEQUENCE LISTING

<110> Academisch Medisch Centrum

<120> Detection of enterovirus RNA and method for reverse
      transcribing RNA

<130> P68624EP00

<140> 04076193.4
<141> 2004-04-19

<160> 7

<170> PatentIn Ver. 2.1

<210> 1
<211> 146
<212> DNA
<213> Artificial Sequence

<220>
<223> Description of Artificial Sequence: armoured RNA

<220>
<221> misc_feature
<222> (1)..(146)

<400> 1
ccctgaatgc ggctaatcct aaccacggaa caggcggtcg cgaaccagtg actggcttgt 60
cgtaacgcgc aagtctgtgc ttgagacgtg cgtggtaacc gtccgtgttt cctgttattt 120
ttatcatggc tgcttatggt gacaat                                      146

<210> 2
<211> 17
<212> DNA
<213> Artificial Sequence

<220>
<223> Description of Artificial Sequence: primer ID#1

<220>
<221> misc_feature
<222> (1)..(17)

<400> 2
ccctgaatgc ggctaat                                                17

<210> 3
<211> 19
<212> DNA
<213> Artificial Sequence

<220>
<223> Description of Artificial Sequence: primer ID#2

<220>
<221> misc_feature
<222> (1)..(19)

<400> 3
attgtcacca taagcagcc                                                    19


<210> 4
<211> 21
<212> DNA
<213> Artificial Sequence

<220>
<223> Description of Artificial Sequence: probe ID#3

<220>
<221> misc_feature
<222> (1)..(21)

<400> 4
gcggaaccga ctactttggg t                                                 21


<210> 5
<211> 21
<212> DNA
<213> Artificial Sequence

<220>
<223> Description of Artificial Sequence: probe ID#4

<220>
<221> misc_feature
<222> (1)..(21)

<400> 5
cttgagacgt gcgtggtaac c                                                 21


<210> 6
<211> 90
<212> DNA
<213> Artificial Sequence

<220>
<223> Description of Artificial Sequence: Ent-hyb-3
      oligonucleotide

<220>
<221> misc_feature
<222> (1)..(90)

<400> 6
ccctgaatgc ggctaatcct aaccacggaa caggcggtcg cgaaccagtg actggtctgt 60
cgtaacgcgc aagtctgtgc ttgagacgtg                                        90

<210> 7
<211> 76
<212> DNA
<213> Artificial Sequence

<220>
<223> Description of Artificial Sequence: Ent-hyb-4
      oligonucleotide

<220>
<221> misc_feature
<222> (1)..(76)

<400> 7
attgtcacca taagcagcca tgataaaaat aacaggaaac acggacggtt accacgcacg 60
tctcaagcac agactt 76